# EUROPEAN PATENT APPLICATION

(11) **EP 3 020 391 A1**
(43) Date of publication of application: **18.05.2016**
(21) Application number: 14822201.1
(22) Date of filing: 08.04.2014
(51) Int. Cl.: A61K 8/64, A61K 8/98, A61Q 19/00

(54) **FUNCTIONAL COSMETIC COMPOSITION COMPRISING GROWTH FACTORS AND AMINO ACIDS**

(30) Priority: 09.07.2013 KR 20130080446
(71) Applicant: Human Biotech. Co. Ltd., Jinju-si, Gyeongsangnam-do 660-844 (KR)
(72) Inventor: LEE, Hyo Jong, Jinju-si Gyeongsangnam-do 660-772 (KR)
(74) Representative: Betten & Resch
(86) International application number: PCT/KR2014/003014
(87) International publication number: WO 2015/005563

(57) **Abstract**

The present invention relates to a functional cosmetic composition containing growth factors and amino acids, and more particularly to a functional cosmetic composition excellent in skin wrinkle improvement, skin resilience improvement, skin moisturization, skin reproduction and skin ageing prevention by containing, as growth factors, epidermal growth factors, fibroblast growth factors and vascular endothelial growth factors, and, as amino acids, proline, glycine and serine. The functional cosmetic composition containing 3 kinds of growth factors and collagen synthesis amino acid precursors and functional cosmetic containing the same have excellent effects on skin wrinkle improvement, skin resilience improvement, skin moisturization, skin reproduction and skin ageing prevention.

## Description

### [Technical Field]

The present invention relates to a functional cosmetic composition containing growth factors and amino acids, and more particularly to a functional cosmetic composition excellent in skin wrinkle improvement, skin resilience improvement, skin moisturization, skin reproduction and skin ageing prevention by containing, as growth factors, epidermal growth factors, fibroblast growth factors and vascular endothelial growth factors, and, as amino acids, proline, glycine and serine.

### [Background Art]

In general, human skin abruptly decreases in terms of functions as ages advance due to changes in lipid composition and content of lipid layer functioning as skin barriers, water content of skin decreases, and skin becomes dried, whereby various skin lesions develop.

Excessive fatigue and stress of modern men, increased amount of ultraviolet due to environmental pollution, and air pollution generate active oxygen high in reactivity, free radicals and peroxide, and the skin is affected thereby to allow biogenic substances (protein, nucleic acid, cell membrane lipid, etc.) to be oxidized, or degenerated, which acts as main causes of skin ageing.

Particularly, collagen composition comprising of skin connective tissue is oxidized to cause the skin to lose its function, resulting in lost skin resilience and excessive formation of wrinkles, and to come to change to senile skin.

Thus, it is important to supplement collagen composition in order to prevent the skin from being aged and to improve the skin, and to this end, researches are being waged on skin-ageing improvement functional materials containing the collagen composition.

Meantime, growth factors are proteins that act to stimulate cell proliferation and differentiation by being bonded to receptors present at surfaces of cells, and have various functions such as, in one instance, acting to stimulate various cells to differentiate, and acting only to a specific type of cell, in other instance. other hand. The growth factors are essential ingredients for stimulating cell proliferation, and the researches on the growth factors have been performed for twenty years, and as a result of the researches, the growth factors are found to adjust the processes of cell proliferation, differentiation and migration.

Among the growth factors, epidermal growth factors, fibroblast growth factors and vascular endothelial growth factors are researched as functional materials with a view to improving the skin ageing.

### [Disclosure of Invention]

### [Technical Subject]

It is an object of the present invention to provide a functional cosmetic composition containing three kinds of growth factors for reproduction of skin cells and amino acid precursors excellent in skin wrinkle improvement, skin resilience improvement, skin moisturization, skin reproduction and skin ageing prevention, and a functional cosmetic using the same.

In one general aspect of the present invention, there is provided a functional cosmetic composition, comprising: as growth factors, epidermal growth factors, fibroblast growth factors and vascular endothelial growth factors, and as amino acids, proline, glycine and serine.

Preferably, but not necessarily, the functional cosmetic composition of claim 1, wherein the growth factors may be derived from human.

Preferably, but not necessarily, the epidermal growth factors, fibroblast growth factors and vascular endothelial growth factors may be respectively included in the composition in a concentration of 0.01ppm ∼ 1.0ppm.

Preferably, but not necessarily, the growth factor may be captured in a nanozome of 30nm ∼ 200nm size.

Preferably, but not necessarily, the proline, glycine and serine may be respectively included in the composition in a concentration of 1ppm∼300ppm.

Preferably, but not necessarily, the functionality may be selected from a group consisting of skin wrinkle improvement, skin resilience improvement, skin moisturization, skin reproduction and skin ageing prevention.

In another general aspect of the present invention, there is provided a functional cosmetic using the above-explained cosmetic composition.

Preferably, but not necessarily, the cosmetic may be selected from a group consisting of skin lotion, skin softener, skin toner, astringent, nutrition toner, epilating supplement, eye cream, nutrition cream, massage cream, cleansing cream, cleansing foam, cleansing water, powder, essence, serum and pack.

### [Advantageous Effects]

The functional cosmetic composition containing three kinds of growth factors and collagen composition amino acid precursors and the functional cosmetic using the same have excellent effects in skin wrinkle improvement, skin resilience improvement, skin moisturization, skin reproduction and skin ageing prevention.

### [Brief Description of Drawings]

FIG. 1a is a photograph of a phase-contrast inverted microscope (100 magnification) after inoculation of human dermal fibroblast cells in DMEM culture medium.
FIG. 1b is a photograph of a phase-contrast inverted microscope (100 magnification) where human dermal fibroblast cells are cultivated and developed in DMEM culture medium.
FIG. 1c is a photograph of a phase-contrast inverted microscope (100 magnification) where human fibroblast cells are cultivated and developed in DMEM culture medium respectively added by human demal fibroblast growth factors, fibroblast growth factors and vascular endothelial growth factors.
FIG. 2 is a graph showing a comparative investigative result of cell proliferation effect by cultivating fibroblast cells in DMEM culture medium respectively added by human demal fibroblast growth factors, fibroblast growth factors and vascular endothelial growth factors according to a first exemplary embodiment.
FIG. 3 is a graph showing an investigative result, by ELISA assay method, of collagen synthesis of human fibroblast cells in DMEM culture medium containing human demal fibroblast growth factors, fibroblast growth factors and vascular endothelial growth factors and proline, glycine and serine as collagen precursors.

### [Best Mode for Carrying out the Invention]

Hereinafter, the present invention will be explained in detail.

The inventors of the present invention carried out researches on cosmetic composition including three kinds of growth factors having a synergy effect to reproduction of skin cells, and ascertained that fibroblast cells proliferation and collagen synthesis are significantly increased when the cosmetic composition is contained by proline, glycine and serine which are collagen biosynthesis precursors, and completed an invention using the same as functional cosmetic composition.

Therefore, the present invention provides a functional cosmetic composition containing epidermal growth factors and fibroblast growth factors, as growth factors, and proline, glycine and serine, as vascular endothelial growth factors and amino acids.

The three kinds of growth factors are preferably derived from human. In an exemplary embodiment, the growth factors may use what is separated and refined from colon bacterium transformed to gene encoding the human-derived epidermal growth factors, fibroblast growth factors and vascular endothelial growth factors.

It is preferable that the epidermal growth factors, fibroblast growth factors and vascular endothelial growth factors be respectively included in the composition in a concentration of 0.01ppm∼1.0ppm. It is difficult to obtain purposeful object when the concentration is less than 0.01ppm, and an issue of expense and stability problem to human may be generated due to excessive use of growth factors when the concentration is more than 1.0ppm.

It is preferable that the growth factor be captured in a nanosome of 30nm ∼ 200nm size. Th growth factor ingredient according to the present invention has disadvantages of skin penetration problem and decreased stability due to being deactivated when left for a long time in room temperature, the disadvantages of which can be solved by nanosome capture. In an exemplary embodiment, the nanosome may be manufactured by a microfludizer using lecithin as raw material, and the growth factors of the present invention may be, respectively or along with, captured inside lecithin particles. A manufacturing method of nanosome may use any method that is well known in the art. It is preferable that a nanosome particle be in 30nm ∼ 200nm size, and when the nanosome particle is less than 30nm size, the skin penetration may be very quickly realized to generate a skin side effect, and when the nanosome particle is greater than 200nm size, the skin penetration is not easily realized, failing to obtain an effect of using the nanosome structure.

It is preferable that the proline, glycine and serine be respectively included in the composition in a concentration of 1ppm∼300ppm. The proline, glycine and serine according to the present invention are amino acid precursors for synthesis of collagen which is an extracellular matrix component, and used for collagen synthesis of cells by reacting with growth factors included in the composition.

The functionality may refer to contribution ability to improvement of skin conditions, e.g., skin wrinkle improvement, skin elasticity improvement, skin moisturization, skin reproduction and skin ageing prevention, but not limited thereto.

The functional cosmetic composition thus explained according to the present invention has a significant effect on fiberblast proliferation and collagen synthesis as described in the following exemplary embodiment, and may be appliable for use as functional cosmetics for improving skin conditions.

Thus, the present invention provides a functional cosmetic using the cosmetic composition according to the description of the present invention.

The cosmetics may be preferably selected from a group consisting of skin lotion, skin softener, skin toner, astringent, nutrition toner, epilating supplement, eye cream, nutrition cream, massage cream, cleansing cream, cleansing foam, cleansing water, powder, essence, serum and pack, but not limited thereto.

The cosmetic composition according to an exemplary embodiment of the present invention may be manufactured by cosmetics well known in the art, and ingredients contained in the cosmetics may include conventionally-used ingredients in addition to 3 kinds of growth factors and collagen precursors. By way of example, the ingredients may include conventional adjuvant and supporting materials (carriers) such as different active ingredients, stabilizers, solubilizers, vitamins, pigments and spiceries.

The cosmetics according to an exemplary embodiment of the present invention may be manufactured by any formulations conventionally manufactured in the art, and may be formulated by solution, suspension, emulsion, paste, gel, cream, lotion, powder, soap, cleansing, oil, powder foundation, emulsion foundation, wax foundation and spray, for example. By way of example, in case of the formulation of the present invention being cream, lotion or gel, oil phase ingredients of adequate oils, surfactants, water, thickeners and stabilizers are mixed to manufacture W/O or O/W type emulsifiable concentrates, and the manufacturing methods thus described are well known to the skilled in the art.

Hereinafter, the present invention will be explained in more details through exemplary embodiments.

### [Exemplary Embodiments]

### First Exemplary Embodiment

In the first exemplary embodiment, human growth factors (EGF, FGF, VEGF) were added to the cell culture medium and tested a growth promotion effect of skin fibroblast.

### (1) Separation of fibroblast from human tissue

Human abdominal dermal tissue was collected, put into 10 cm sterilized beaker, and washed with same volume (1:1) of phosphorylation buffer solution (D-PBS). 20 ml of D-PBS was put into a clean bench, the tissue is held by one hand with forceps and the skin tissue was cut to small pieces of 2mm size using a surgery knife. The cut tissue and solution are retrieved, and collected in several 50ml centrifugal conical tubes, 20ml each. A same amount of 0.15 % collagenase type-1 solution (Invitrogen) was inputted, and reacted for 1-2 hours at 37 °C, which was then washed using D-PBS and centrifuged at 400 x g for 5 minutes. The supernatant was removed. The pellet remaining at a floor was added with 10-40 ml of D-PBS, and the settled solidified cells were dissolved to allow passing through 70-100 mm mesh filter, whereby solids were removed. The retrieved supernatant were put into 10 ml of DMEM+10% FBS culture medium, centrifuged at 400 x g for 5 minutes to remove the supernatant and wash the cells. This wash was repeated twice to cast away the supernatant and obtain only the cells.

### (2) Manufacturing of culture medium

The culture medium used in the present experiment, used as a treatment plot, is added by, as growth factors, 0.5 PPM each for human Epidermal growth factors, human fibroblast growth factors, human vascular endothelial growth factors to DMEM (Dulbecco's Modified Eagle's Medium) medium containing penicillin (100 IU/mL), streptomycin (100 g/mL), and 10% FBS(fetal bovine serum.

### (3) Culture of human fibroblast

The human fiberblast was dispensed in a 12-well plate at a density of 1 x 10⁵ cells/well, and treated independently in a DMEM(Dulbecco's Modified Eagle's Medium, Gibco) culture medium containing penicillin (100 IU/mL), streptomycin (100 g/mL), anti-fungal substances and 10% FBS(fetal bovine serum) or in a DMEM culture medium added by human Epidermal growth factors, human fibroblast growth factors, and human vascular endothelial growth factors, and cultured in an incubator including 5% carbon dioxide at 37°C for 5 days.

### (4) Development efficacy tests of human fiberblast

The culture medium was removed 5 days after culture, treated with 2-5 ml of 0.05% trypsin solution, reacted for 5 minutes to separate the cells, washed at least twice using centrifugal method, and only cells were collected. The measurement of the number of cells and viability tests were conducted in such a manner that 0.1ml of cell suspension and equal amount of 0.2% trypan blue(SIGMA) are mixed to obtain a percentage of total cells by counting dyed cells and undyed cells under a microscopic view using a hemocytometer.

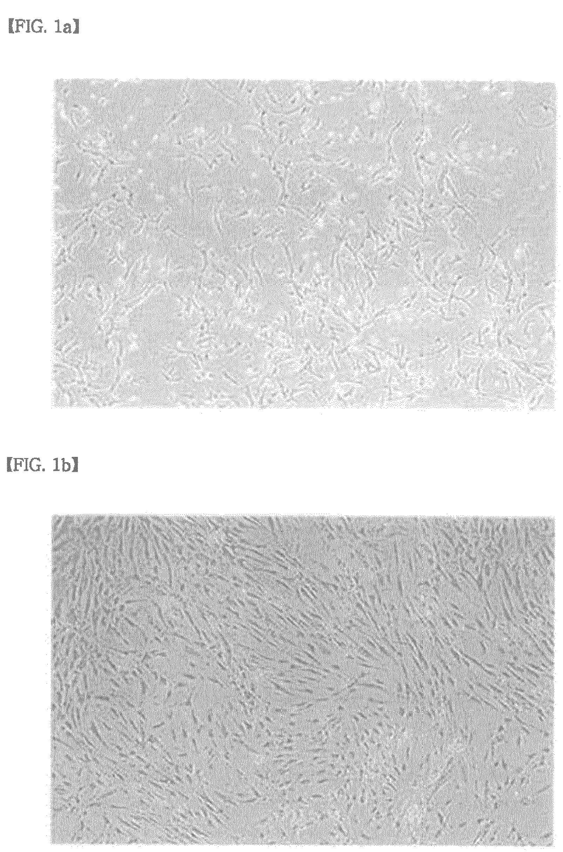

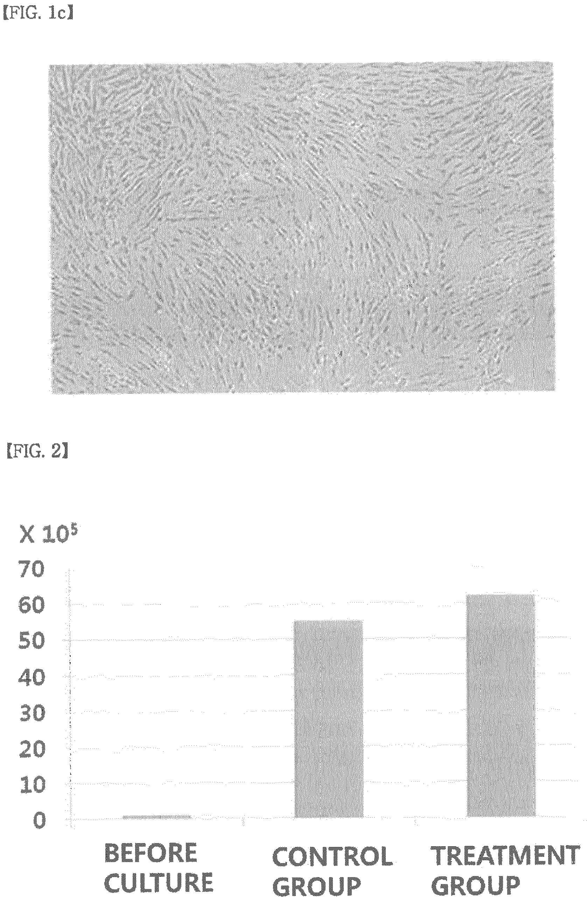

FIGS. 1a to 1c are microscopic observant views of aspects of cell proliferations after inoculation of human fiberblast into an incubator at a density of 1 x 10⁵ cells/well (FIG. 1a), and after incubation in a DMEM culture medium not added by human growth factors (EGF, FGF, VEGF) for 5 days (FIG. 1b) and incubation in DEEM culture medium added by human growth factors for 5 days (FIG. 1c). The fiberblast were proliferated 62 times in a treatment group added by growth factors, while the fiberblast were proliferated 55 times in a control group not added by growth factors, whereby it was confirmed that addition of growth factors showed a significantly high cell growth efficiency (FIG. 2).

### Second Exemplary Embodiment

A second exemplary embodiment confirmed the collagen formation potential of human fiberblast in a DMEM culture medium added by human growth factors and collagen precursors.

### (1) Separation of fiberblast from human tissue

Human abdominal dermal tissue was collected, put into 10 cm sterilized beaker, and washed with same volume (1:1) of phosphorylation buffer solution (D-PBS). 20 ml of D-PBS was put into a clean bench, the tissue is held by one hand with forceps and the skin tissue was cut to small pieces of 2mm size using a surgery knife. The cut tissue and solution are retrieved, and collected in several 50ml centrifugal conical tubes, 20ml each. A same amount of 0.15 % collagenase type-1 solution (Invitrogen) was inputted, and reacted for 1-2 hours at 37 °C, which was then washed using D-PBS and centrifuged at 400 x g for 5 minutes. The supernatant was removed. The pellet remaining at a floor was added with 10-40 ml of D-PBS, and the settled solidified cells were dissolved to allow passing through 70-100 mm mesh filter, whereby solids were removed. The retrieved supernatant were put into 10 ml of DMEM+10% FBS culture medium, centrifuged at 400 x g for 5 minutes to remove the supernatant and wash the cells. This wash was repeated twice to cast away the supernatant and obtain only the cells.

### (2) Manufacturing of culture medium

The culture medium used in the present experiment, used as a treatment plot, is added by, as growth factors, 0.5 PPM each for human Epidermal growth factors, human fibroblast growth factors, human vascular endothelial growth factors to DMEM (Dulbecco's Modified Eagle's Medium) medium containing penicillin (100 IU/mL), streptomycin (100 g/mL), and 10% FBS(fetal bovine serum), and as collagen precursors, 100 PPM each of proline, glycine and serine.

### (3) Culture of human fibroblast

The human fiberblast was dispensed in a 12-well plate at a density of 1 x 10⁵ cells/well, and treated independently in a DMEM(Dulbecco's Modified Eagle's Medium, Gibco) culture medium containing penicillin (100 IU/mL), streptomycin (100 g/mL), anti-fungal substances and 10% FBS(fetal bovine serum) or in a DMEM culture medium added by human Epidermal growth factors, human fibroblast growth factors, and human vascular endothelial growth factors, and in a DMEM culture medium added, as collagen precursors, by 100 PPM each of proline, glycine and serine, and cultured in an incubator including 5% carbon dioxide at 37°C for 5 days.

### (4) Measurement of collagen quantity inside reagent

ELISA (Sircol soluble collagen assay kit) method was used to measure the collagen quantity. That is, a sample of 1.0 ml was placed into 1.5 ml conical micro tubes, and 100 µl of acid neutralising reagent was placed in which 200µl of isolation and concentration reagent 200 µl was mixed. The reagent was reacted overnight in ice water, and centrifuged at 12,000 rpm for 10 minutes. The supernatant was removed and added by 1.0ml of Sircol dye reagent Then, the reagent was measured at 450nm using an ELISA reader. Standard solution was prepared in a manner that water was poured into collagen standard and melted to be respectively diluted to become 0, 10, 20, 30, 40 and 50 µg/ml.

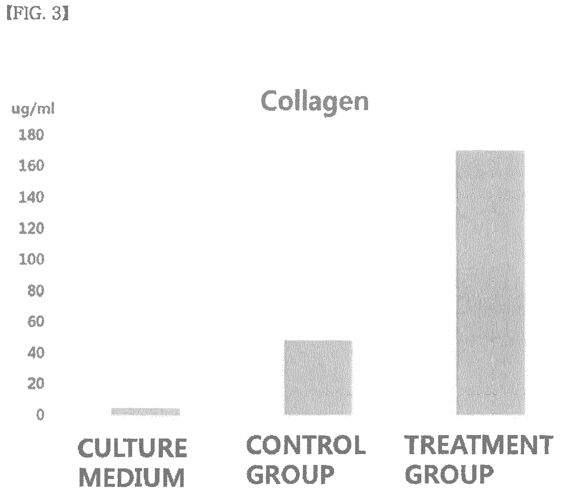

FIG. 3 illustrates a measurement result, by ELISA method, of collagen production after inoculating human fiberblast in a 75T flask at a density of 1 x 10⁶ cells/well, and cultured for 5 days. The collagen production was 48 µg/ml in a culture medium (control group) in which the human growth factors (EGF, FGF, VEGF) and proline and serine as collagen precursors were not added, and the collagen production was 170 µg/ml in a culture medium (treatment group) in which the human growth factors (EGF, FGF, VEGF) and proline, glycine and serine as collagen precursors were added. Although the collagen production in the treatment group added by growth factors and precursors increased by 34 times during culture time, the collagen production increased by 9.6 times in the control group not added by growth factors and precursors, the result of which showed that the addition of growth factors and precursors accomplished a significantly high collagen synthesis ability.

### [INDUSTRIAL APPLICABILITY]

As noted from the results of FIGS. 2 and 3, the functional cosmetic composition according to the methods in the exemplary embodiments of the present invention can promote proliferation of fiberblast, and increase the collagen synthesis, such that the methods can be effectively applicable to functional cosmetics targeted to skin anti-ageing and anti-wrinkle effects.

## Claims

1. A functional cosmetic composition, the functional cosmetic composition comprising: as growth factors, epidermal growth factors, fibroblast growth factors and vascular endothelial growth factors, and as amino acids, proline, glycine and serine.

2. The functional cosmetic composition of claim 1, wherein the growth factors are derived from human.

3. The functional cosmetic composition of claim 1. wherein the epidermal growth factors, fibroblast growth factors and vascular endothelial growth factors are respectively included in the composition in a concentration of 0.01ppm∼ 1.0ppm.

4. The functional cosmetic composition of claim 1, wherein the growth factor is captured in a nanozome of 30nm ∼ 200nm size.

5. The functional cosmetic composition of claim 1, wherein the proline, glycine and serine are respectively included in the composition in a concentration of 1ppm~300ppm.

6. The functional cosmetic composition of claim 1, wherein the functionality is selected from a group consisting of skin wrinkle improvement, skin resilience improvement, skin moisturization, skin reproduction and skin ageing prevention.

7. A functional cosmetic using the above-explained cosmetic composition of claim 1.

8. The functional cosmetic of claim 7, wherein the cosmetic is selected from a group consisting of skin lotion, skin softener, skin toner, astringent, nutrition toner, epilating supplement, eye cream, nutrition cream, massage cream, cleansing cream, cleansing foam, cleansing water, powder, essence, serum and pack.
